# EUROPEAN PATENT SPECIFICATION

(11) **EP 2 011 488 B1**
(45) Date of publication and mention of the grant of the patent: **22.08.2012**
(21) Application number: 07720334.7
(22) Date of filing: 06.03.2007
(51) Int. Cl.: A61K 9/70, A61K 47/30, A61K 31/135, A61P 25/00

(54) **TRANSDERMAL PATCH CONTAINING RASAGILINE FOR TREATMENT OR PROPHYLAXIS OF NERVOUS SYSTEM DISEASE AND ITS PREPARATION PROCESS**
TRANSDERMALES PFLASTER MIT RASAGILIN ZUR BEHANDLUNG ODER PROPHYLAXE VON ERKRANKUNGEN DES NERVENSYSTEMS UND SEIN HERSTELLUNGSVERFAHREN
TIMBRE TRANSDERMIQUE CONTENANT DE LA RASAGILINE UTILISE DANS LE TRAITEMENT OU LA PREVENTION DE MALADIES DU SYSTEME NERVEUX ET PROCEDE DE PREPARATION ASSOCIE

(30) Priority: 06.03.2006 CN 200610054110
(43) Date of publication of application: 07.01.2009
(73) Proprietor: Chongqing Pharmaceutical Research Institute Co., Ltd., Chongqing 400061 (CN); Shanghai Fosun Pharmaceutical (Group) Co., Ltd., Shanghai 200010 (CN)
(72) Inventor: LIN, Jialiang, Chongqing 400061 (CN); XIAO, Jinmai, Chongqing 400061 (CN); DENG, Jie, Chongqing 400061 (CN)
(74) Representative: Maiwald, Walter
(86) International application number: PCT/CN2007/000713
(87) International publication number: WO 2007/101400

(56) References cited:
- EP-A2- 1 561 461
- WO-A1-2005/072705
- WO-A2-01/34172
- US-A- 5 462 746
- US-A- 5 972 376
- US-A1- 2003 087 814
- US-A1- 2005 079 206

## Description

### FIELD OF INVENTION

The present invention pertains to the field of pharmaceutical preparations, and particularly relates to a transdermal patch containing rasagiline for treatment or prophylaxis of nervous system diseases and to a method for preparing the same. The transdermal patch comprises a backing layer chemically inert to substrate ingredients, a substrate layer containing an effective amount of rasagiline or a pharmaceutically acceptable salt thereof, and a protective foil or film to be peeled off before use.

### BACKGROUND OF THE INVENTION

Rasagiline, a selective inhibitor of monoamine oxidase-B, is used for the treatment of central nervous system diseases, such as Parkinson's disease (PD), depression, etc., and has gone on sale in Europe. The chemical structure of rasagiline is as follow:

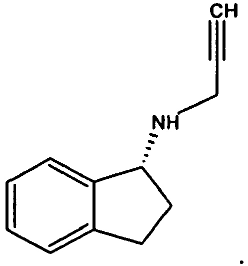

It is well known that Parkinson's disease is a consequence of the regression of dopaminergic nerve in nerve centre and the reduction of release of neurotransmitter dopamine. The insufficient release of dopamine transmitter may result in muscle control dysfunction, tremor, muscle rigor, holotonia, joint motion dysfunction in patients. Some patients even are difficult in turning over, may move retardedly, awkwardly and asynergically (bradypragia), and cannot complete fine activities such as tying shoelace, buckling button, etc. Some patients in severe condition even cannot stand and walk and may have abnormal posture.

Pharmacotherapies of Parkinson's disease mainly focus on direct or indirect elevation of intracerebral dopamine level. Principal drugs for treatment of Parkinson's disease are levodopa, dopamine agonists, amantadine, anticholinergic agents, etc. During the later 1960's, levodopa (L-dopa) as a substitute of neurotransmitter dopamine started to be used for treatment of Parkinson's disease, and is still a principle drug nowadays. Since levodopa is rapidly decarboxylated extracerebrally and converted into dopamine, the dosage of levodopa is relatively large and adverse effects occur frequently. Thus, levodopa usually is administered in combination with a decarboxylase inhibitor in order to improve the ability of levodopa in passing through blood-brain barrier. Another type of drugs for treatment of Parkinson's disease is dopamine agonists, including bromocriptine and pergolide mesylate, which inhibit enzymes degrading dopamine to elevate intracerebral dopamine level. Dopamine agonists usually have a relatively long half-life, which can directly act on receptor, and can reduce the administration frequency and dose of levodopa (Li Tianqing, Science and Technology Development Center of the Chinese Pharmaceutical Association, Market Analysis of Drugs for Treatment of Parkinson's Disease, the website of the Chinese Pharmaceutical Association).

Rasagiline is an irreversible selective monoamine oxidase-B (MAOB) inhibitor. In a double-blind randomized clinical test, the effectiveness of rasagiline on early PD was observed (Arch Neurol, 2004;61:561~566). In the test, 404 patients with early PD were administered with rasagiline (1mg/d or 2mg/d) for 1 year, or with rasagiline for 6 months and then placebo for 6 months. The main evaluation index was the score change of unified Parkinson's disease rating scale (UPDRS). The results indicated that 371 patients completed the clinical test, the mean UPDRS score of the patients administered with rasagiline 2mg/d for 1 year was 2.29 units lower than that of the patients administered with placebo in the last 6 months, and 1.82 units lower than that of the patients administered with rasagiline 1mg/d for 1 year. The researchers concluded that the patients administered with rasagiline (1mg/d or 2mg/d) for 1 year exhibited a lower degree of hypofunction than the patients administered with placebo. Although rasagiline is similar to other antiparkinsonian drugs, it has a lower incidence rate of toxic and side-effects (mainly including insomnia, nausea and illusion).

Since rasagiline has a relatively strong potency, and the eating during oral administration may reduce its blood peak concentration by 60%, moreover most parkinsonians are difficult in walking and oral taking, it is necessary to formulate rasagiline for transdermal administration so that rasagiline may be administered every other day or more. In addition, although rasagiline is a selective monoamine oxidase-B (MAOB) inhibitor, it may simultaneously inhibit both MAO-A and MAO-B at a relatively high blood concentration, so that a so-called "Cheese Reaction" may occur, i.e., when an irreversible non-selective drug is administered to a patient, if the patient takes a tyramine- or dopamine-containing drug or food, the patent may suffer hypertensive crisis. Since cooked cheese contains a large amount of tyramine, this reaction is called "Cheese Reaction". Some reports disclosed that when some patients were orally administered with rasagiline 10mg/d in combination with levodopa, their therapy may be discontinued due to adverse cardiovascular reactions such as hypertension and posture hypertension. These adverse reactions may be associated with the non-selectivity to MAO-A and MAO-B. A transdermal patch containing rasagiline may lead to a moderate absorption and can avoid or reduce "Cheese Reaction", and once a patient feels unwell due to "Cheese Reaction", the patch can be peeled off immediately to avoid more severe results.

US2004013620 described a transdermal therapeutic system comprising rasagiline as active ingredient, characterized in that a transdermal penetration enhancer of the following formula is used:

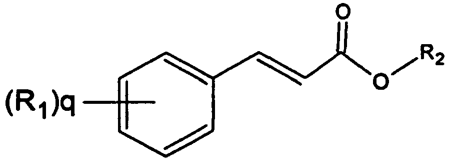

However, the enhancement effects of this transdermal penetration enhancer on the transdermal penetration of rasagiline are not very good, and the used spray transdermal absorption results in inconvenience of administration. Therefore, a patch having convenience in administration and good transdermal absorption effects is still in need to provide more options for patients.

### SUMMARY OF THE INVENTION

One embodiment of the present invention provides a transdermal patch of rasagiline for treatment or prophylaxis of nervous system diseases, wherein the patch comprises an inert backing layer chemically inert to substrate ingredients, a substrate layer comprising rasagiline or a pharmaceutically acceptable salt thereof, and a protective layer to be peeled off before use. The substrate layer is a drug-carrying reservoir comprising an organic polymer material and an inorganic or organic material as regulator, in which the reservoir comprises rasagiline. The substrate layer further comprises one or more substances for enhancing the transdermal absorption of rasagiline. If appropriate, the substrate layer may further comprises a controlled release substrate layer and/or an adhesive layer, in which the controlled release substrate layer may also comprise rasagiline as active ingredient, and the adhesive layer may or may not comprise rasagiline as active ingredient. If the drug reservoir substrate layer or the controlled release substrate layer has an appropriate adhesiveness, the adhesive layer as a separate layer may be unnecessary.

Another embodiment of the present invention is to provide a use of the transdermal patch containing rasagiline according to the present invention for the treatment or prophylaxis of Parkinson's disease, Alzheimer's disease, depression, hyperactive child syndrome, restless leg syndrome, multiple sclerosis and abstinence syndrome.

In the above rasagiline transdermal patch, rasagiline or a pharmaceutically acceptable salt thereof has an effective amount of 0.01mg/cm²~50mg/cm² in the substrate layer, based on the free base of rasagiline.

The rasagiline transdermal patch of the present invention has an administration area of about 1cm²~50cm² during a transdermal therapy.

In the rasagiline transdermal patch of the present invention, rasagiline or a pharmaceutically acceptable salt thereof in the substrate layer is present in the form of, free base.

In the rasagiline transdermal patch of the present invention, the pharmaceutically acceptable salt of rasagiline includes hydrochloride, mesylate, esylate or sulfate, preferably mesylate.

In the rasagiline transdermal patch of the present invention, the organic polymer material in the substrate layer includes at least one type of the following organic polymer materials: polyacrylates and derivatives thereof, polyvinylpyrrolidone and derivatives thereof, polyvinyl alcohol and derivatives thereof, silicone polymers and derivatives thereof, polyisobutylene and derivatives thereof, ethylene-vinyl acetate copolymers and derivatives thereof, or carbopol and derivatives thereof.

In the rasagiline transdermal patch of the present invention, the inorganic or organic material in the substrate layer as regulator includes one or more of the following substances: water, sodium hydroxide, sodium phosphate, sodium hydrogen phosphate, sodium dihydrogen phosphate, sodium carbonate, sodium hydrogen carbonate, sodium chloride, sodium sulfate, sodium hydrogen sulfate, potassium hydroxide, potassium phosphate, potassium hydrogen phosphate, potassium dihydrogen phosphate, potassium carbonate, potassium hydrogen carbonate, potassium chloride, potassium sulfate, potassium hydrogen sulfate, calcium hydroxide, calcium phosphate, calcium hydrogen phosphate, calcium carbonate, calcium chloride, calcium sulfate, phosphoric acid, hydrochloric acid, sulfuric acid, silicon dioxide, silicic acid, sodium metasilicate, potassium metasilicate, sodium trisilicate, potassium trisilicate, titanium dioxide, ethanediamine, triethylamine, triethanolamine, methanol, ethanol, propanol, propylene glycol, glycerol, butanol, chloroform, dichloromethane, tetrachloromethane, urea, petroleum ether, polyvidone, polyethylene glycol, long-chain fatty alcohols, oils or lipids.

In the rasagiline transdermal patch of the present invention, the substance for enhancing the transdermal absorption of rasagiline (rasagiline transdermal penetration enhancer) in the substrate includes at least one of the following substances: ethanol, propylene glycol, oleic acid, ocenol, linoleic acid, laurinol, lauric acid, isopropyl myristate, laurocapram (azone,) and homologues thereof, or terpenes. The weight ratio of rasagiline to the transdermal penetration enhancer is 1:1~1:5.

Further embodiments of the present invention provide two methods for preparing the rasagiline transdermal patch.

### Method 1:

A method for preparing a rasagiline transdermal patch, wherein the method comprises the following steps:
a) Blending an effective amount of rasagiline or a pharmaceutically acceptable salt thereof into an organic polymer material pre-heated to 50°C~200°C;
b) Adding a regulator and a substance for enhancing transdermal adsorption, mixing uniformly to obtain a mixture substrate so that the rasagiline or a pharmaceutically acceptable salt thereof in the substrate is present in the form of free base and
c) Smearing the mixture substrate on an inert backing layer to form a uniform thin film, and sticking thereon a protective layer to obtain the patch.

### Method 2:

A method for preparing a rasagiline transdermal patch, wherein the method comprises the following steps:
a) Adding an effective amount of rasagiline or a pharmaceutically acceptable salt thereof to a solvent with a desired volatility, adding thereto and fully swelling an organic polymer material;
b) Adding thereto simultaneously or in order a regulator and a substance for enhancing transdermal adsorption to obtain a mixture substrate so that the rasagiline or a pharmaceutically acceptable salt thereof is present in form free base and
c) Smearing the mixture substrate on an inert backing layer to form a uniform thin film, and sticking thereon a protective layer to obtain the patch.

In the methods of the present invention for preparing rasagiline transdermal patch, the step c) may further comprise repeating the smearing by any way to obtain a multilayer thin film substrate.

If appropriate, the multilayer thin film substrate may further comprise a controlled release drug layer and/or an adhesive layer.

The rasagiline transdermal patch of the present invention is used for the treatment or prophylaxis of a nervous system disease selected from Parkinson's disease, Alzheimer's disease, depression, hyperactive child syndrome, restless leg syndrome, multiple sclerosis and abstinence syndrome.

### DETAILED DESCRIPTION OF THE INVENTION

The transdermal administration system of rasagiline or a pharmaceutically acceptable salt thereof in the present invention is a patch, which has transdermal effects better than those described in US2004013620, and the used transdermal penetration enhancers are more common and available.

Therefore, it is particularly cared in the present invention that the active substance rasagiline or a pharmaceutically acceptable salt thereof can be most effectively incorporated into the system and transferred through skin.

One embodiment of the present invention provides a transdermal patch of rasagiline for treatment or prophylaxis of nervous system diseases and a method for preparing the same, wherein the patch comprises an inert backing layer chemically inert to substrate ingredients, a substrate layer comprising an effective amount of rasagiline or a pharmaceutically acceptable salt thereof, and a protective layer to be peeled off before use. The substrate is a drug reservoir and/or an adhesive body comprising a viscous and/or non-viscous organic polymer material and an inorganic or organic material as filler, and the substrate layer has a plurality of micro-reservoirs containing rasagiline. The substrate layer further comprises one or more substances for enhancing the transdermal absorption of rasagiline (a selective monoamine oxidase inhibitor). The organic polymer material is used as a substrate material for rasagiline or a pharmaceutically acceptable salt thereof, and is preferably selected from polyacrylates and derivatives thereof, polyvinyl alcohol and derivatives thereof, polyvinylpyrrolidone and derivatives thereof, silicone polymers and derivatives thereof, polyisobutylene and derivatives thereof, ethylene-vinyl acetate copolymers and derivatives thereof, and carbopol and derivatives thereof. The organic polymer material may be self-adhesive in some extent for facilitating bonding on skin.

The above substrate layer comprises a plurality of micro-reservoirs so that the release of rasagiline can be sustained for 2-3 days or more (e.g., one week), to maintain an effective blood concentration in vivo.

If appropriate, the above substrate layer may further comprise a controlled release layer and/or an adhesive layer. "If appropriate" this means it is determined upon the viscosity properties and controlled drug release rate of different polymers used in the substrate layer.

The inert backing layer (underlay) can be a foil or a film of polyethylene or polypropylene or a non-woven fabric. The protective layer (antistick layer) can also be a foil, or a complex film formed by materials such as polyethylene or polypropylene or polycarbonate, etc, or a thick slick paper pretreated with paraffin or methylsilicone oil.

In the simplest embodiment, these substrate systems can be a single phase substrate comprising a backing layer, an organic polymer substrate having a desired self-adhesiveness and comprising an active substance, and a protective layer to be peeled off before use. A relatively complex embodiment comprises a multilayer substrate, and these substrate layers may also comprise a non-adhesive layer and a control film. The substrate can be filled with an inert filler to improve cohesiveness among ingredients.

In the substrate, the active substance rasagiline is present in the form of free base. In any of these forms, the active ingredient is saturated relative to the substrate system and has the maximum thermodynamic activity. Since the free groups of polymer notably affect the release of the active ingredient, some organic or inorganic regulator must be added in order to maintain the active ingredient in desired form on the one hand and to regulate the release of the active ingredient on the other hand. In the meantime, the regulator may further change the physicochemical properties of the substrate to facilitate the Smearing of the substrate and the release of the active substance from the substrate. Since rasagiline is usually present in form of mesylate or hydrochloride and has a relatively high dissociation tendency, in the following examples 1~5, aqueous NaOH solution is used to adjust PH value to 7.5, so that on the one hand, rasagiline can be present in the form of free base to a great extent under this PH condition to enhance adsorption, and on the other hand, this PH is not too high to irritate skin. In addition, NaOH can be readily available and thus is preferred. In example 6, triethanolamine is used as a regulator to adjust PH value to 8.5, and irritation is not severe due to the insulation of silicone film. Although NaOH and other alkaline substances may also be used, triethanolamine is preferred because it has moderate alkality and the obtained gel is uniform and free of air bubbles and granules.

Rasagiline has to penetrate skin in the form of free base.

Stratum corneum is a main barrier for transdermal absorption, so some substances capable of softening stratum corneum and enhancing lipid fluidity in skin tissue can enhance the transdermal effects of active ingredients. Some organic solvents, fatty acids, fatty alcohols, laurocapram, surfactants, keratolytic humectant, terpenes and plant volatile oils, cyclodextrins can enhance the transdermal effects of active ingredients. In the present invention, ethanol, propylene glycol, oleic acid, ocenol, linoleic acid, laurinol, lauric acid, isopropyl myristate, azones or terpenes, or any mixtures thereof, in which azones are preferably laurocapram, and terpenes are preferably menthol. According to some tests, when rasagiline : the substance enhancing transdermal absorption = 1:1~1:5(W/W), rasagiline has better transdermal absorption, in which when rasagiline : the substance enhancing transdermal absorption < 1:3(W/W), the change of the amount of the substance enhancing transdermal absorption did not significantly affect the absorption of rasagiline, and when rasagiline : the substance enhancing transdermal absorption reached 1:5 (W/W), the absorption of rasagiline was elevated significantly. However, an extremely high amount of the substance enhancing transdermal absorption may cause side-effects. It is concluded from a large number of experiments that many factors may affect the transdermal effects, the order of these factors in accordance with their influence extents is: substrate > presence of penetration enhancer > formation of free base of principal agent > kind of penetration enhancer. When hydrophilic polyvinyl alcohol (PVA124) is used as drug-carrying layer, the transdermal effects are significantly higher than that of polyacrylic resins, polyisobutylene, silicone polymers. When the same substrate is used, the presence of penetration enhancer and the formation of free base further improve the transdermal effects. As for the kind of transdermal penetration enhancer, although it also influences transdermal effects, the influence extent is not notable. The reason is that the physiochemical interaction between the substrate and rasagiline plays an important role in the release of rasagiline. When hydrophilic polyvinyl alcohol (PVA124) is used as drug-carrying layer in rasagiline patch, it absorbs moisture from the skin and the environment and forms a saturation solution of rasagiline, thereby enhancing the release of rasagiline and greatly elevating the penetration amount of rasagiline.

The detailed description of the preparation of patch is given in the following examples. The penetration properties of the finished patches are studied by using Franz cell (effective reception area: 1.28cm²; volume: 7mL) and skin of abdomen of hairless mice, and the results show that all patches can transdermally provide sufficient amount of rasagiline to fulfill systemic action.

The rasagiline transdermal patch of the present invention can be administered with one patch every 2 -3 days or more, such as one patch every week.

### BRIEF DESCRIPTION OF THE DRAWINGS

Fig.1 shows the cross section views of the patches of Examples 1, 2, 3, 4 and 5.
Fig.2 shows the cross section view of the patch of Example 6.
Fig.3 shows the cross section view of the patches of Examples 7 and 9.
Fig. 4 shows the plane view of the patches of Examples 7 and 9.
Fig.5 shows the cross section view of the patch of Example 8.
Fig.6 shows the plane view of the patch of Example 8.
Fig.7 shows the cross section view of the path of Example 10.
Fig.8 shows the plane view of the patch of Example 10.
Fig.9 shows the relation curve diagram of the time-cumulative penetration amount of rasagiline for patches with high, middle and low oleic acid levels in Example 1.
Fig.10 shows the relation curve diagram of the time-cumulative penetration amount of rasagiline for patches with high, middle and low linoleic acid levels in Example 2.
Fig.11 shows the relation curve diagram of the time-cumulative penetration amount of rasagiline for patches containing oleic acid and propylene glycol in Example 3.
Fig.12 shows the relation curve diagram of the time-cumulative penetration amount of rasagiline for patches with high, middle and low isopropyl myristate levels in Example 4.
Fig.13 shows the relation curve diagram of the time-cumulative penetration amount of rasagiline for patches with high, middle and low laurocapram levels in Example 5.
Fig.14 shows the relation curve diagram of the time-cumulative penetration amount of rasagiline for patches with high, middle and low laurocapram levels in Example 6.
Fig.15 shows the relation curve diagram of the time-cumulative penetration amount of rasagiline for patches with high, middle and low menthol levels in Example 7.
Fig. 16 shows the relation curve diagram of the time-cumulative penetration amount of rasagiline for patches with high, middle and low isopropyl myristate levels in Example 8.
Fig.17 shows the relation curve diagram of the time-cumulative penetration amount of rasagiline for patches with high, middle and low isopropyl myristate levels in Example 9.
Fig.18 shows the relation curve diagram of the time-cumulative penetration amount of rasagiline for patches containing isopropyl myristate in Example 10.

### SPECIFIC MODELS FOR CARRYING OUT THE INVENTION

The following examples are used for further illustrating the present invention, not for restricting the scope of the present invention.

### Example 1: Rasagiline-containing monolayer polyacrylate substrate patches, using oleic acid as transdermal penetration enhancer.

In chloroform, 50g 50%(w/w) Eudragit E100 solution was added to 250g polyacrylate adhesive fully swollen in aqueous solution, then 50g oleic acid (low oleic acid level) was added, and stirred uniformly to obtain a solution.

50g rasagiline was dissolved in 200mL anhydrous ethanol at 50°C~80°C, and then was added to the above solution under stirring. 1 mol/L NaOH aqueous solution was added slowly under stirring until the PH value was 7.5. After the obtained mixture was stirred uniformly, it was smeared on a medical non-woven fabric by using an appropriate scraper, and the thickness of its wet film was adjusted so that a weight of 60g/m² was obtained after it was dried at 100°C for 60min.

The dried substrate thin film was then covered with a polyester film having a thickness of 23µm, and cut to form the finished patches.

The amount of the added oleic acid was 150g (middle level oleic acid) or 250g (high level oleic acid), and all other steps were the same as above described.

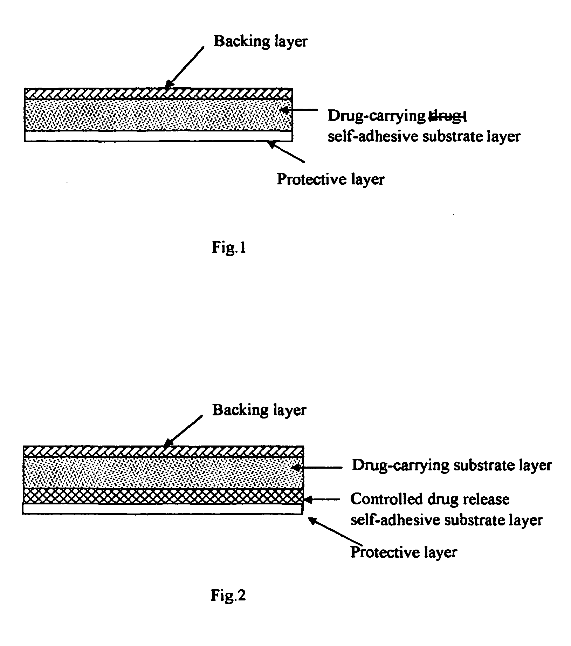

The cross section views of the obtained rasagiline transdermal patches with high, middle and low oleic acid levels are shown in Fig.1.

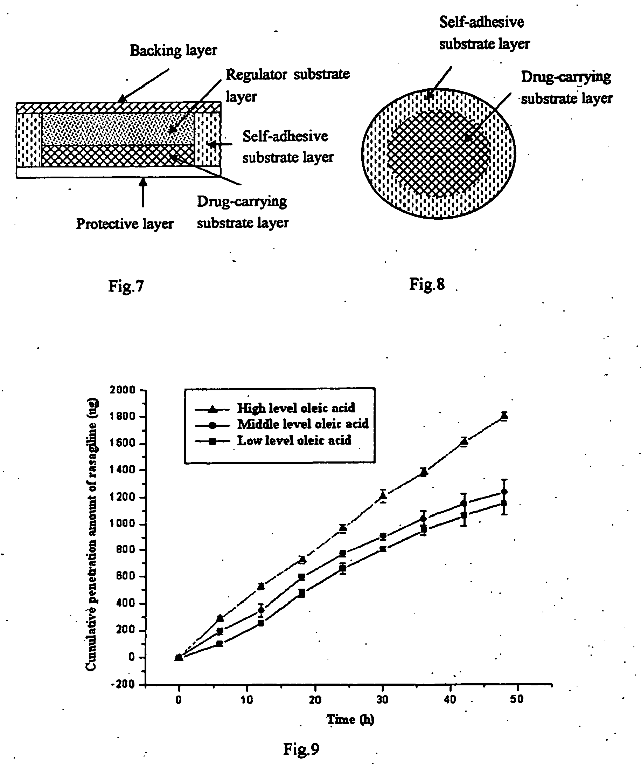

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 1, 2 and 3. Table 1 gives the time-cumulative amount data of rasagiline patch with low level of oleic acid; Table 2 gives the time-cumulative amount data of rasagiline patch with middle level of oleic acid; Table 3 gives the time-cumulative amount data of rasagiline patch with high level of oleic acid. The relation curve diagram of time-cumulative amount of rasagiline is shown in Fig.9.

It can be seen that the cumulative amounts for all patches were over 1000µg at 48h. The patches with high level of oleic acid had a mean cumulative amount of 1786µg at 48h. Thus, this kind of patches can transdermally provide a sufficient amount to achieve systemic effects. The patches with high level of oleic acid had a steeper time-cumulative amount curve, which indicated that their penetration rate was higher.

**Table 1. Time-cumulative penetration amount data of rasagiline patches with low level of oleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 120 | 87 | 100 | 93 | 100 | 14.35 |
| 12 | 260 | 246 | 231 | 268 | 251.3 | 16.28 |
| 18 | 497 | 437 | 457 | 487 | 469.5 | 27.54 |
| 24 | 702 | 607 | 641 | 668 | 654.5 | 40.32 |
| 30 | 825 | 781 | 799 | 796 | 800.3 | 18.28 |
| 36 | 962 | 922 | 910 | 993 | 946.8 | 38.01 |
| 42 | 1130 | 1033 | 958 | 1102 | 1055.8 | 76.87 |
| 48 | 1210 | 1162 | 1024 | 1183 | 1144.8 | 82.86 |

**Table 2. Time-cumulative penetration amount data of rasagiline patches with middle level of oleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 198 | 190 | 168 | 220 | 194 | 21.48 |
| 12 | 321 | 358 | 301 | 402 | 345.5 | 44.46 |
| 18 | 572 | 588 | 579 | 623 | 590.5 | 22.63 |
| 24 | 762 | 778 | 744 | 795 | 769.75 | 21.82 |
| 30 | 906 | 890 | 868 | 926 | 897.5 | 24.57 |
| 36 | 1022 | 1014 | 987 | 1115 | 1034.5 | 55.72 |
| 42 | 1185 | 1144 | 1036 | 1211 | 1144 | 77.10 |
| 48 | 1277 | 1263 | 1088 | 1285 | 1228.25 | 93.94 |

**Table 3. Time-cumulative penetration amount data of rasagiline patches with high level of oleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 308 | 269 | 299 | 267 | 285.75 | 20.84 |
| 12 | 543 | 509 | 537 | 498 | 521.75 | 21.69 |
| 18 | 752 | 715 | 746 | 687 | 725 | 30.08 |
| 24 | 990 | 940 | 986 | 930 | 961.5 | 30.91 |
| 30 | 1255 | 1180 | 1228 | 1146 | 1202.25 | 48.66 |
| 36 | 1405 | 1378 | 1387 | 1340 | 1377.5 | 27.40 |
| 42 | 1636 | 1596 | 1607 | 1553 | 1598 | 34.42 |
| 48 | 1820 | 1760 | 1802 | 1762 | 1786 | 29.80 |

### Example 2: Rasagiline-containing monolayer polyacrylate substrate patches, using linoleic acid as transdermal penetration enhancer.

In chloroform, 50g 50%(w/w) Eudragit E100 solution was added to 250g polyacrylate adhesive fully swollen in aqueous solution, then 50g linoleic acid (low linoleic acid level) was added, and stirred uniformly.

50g rasagiline was dissolved in 200mL anhydrous ethanol at 50°C~80°C, and then added to the above solution under stirring. 1mol/L NaOH aqueous solution was added slowly under stirring until the PH value was 7.5. After the obtained mixture was stirred uniformly, it was smeared on a medical non-woven fabric by using an appropriate scraper, and the thickness of its wet film was adjusted so that a weight of 60g/m² was obtained after it was dried at 100°C for 60min.

The dried substrate thin film was then covered with a polyester film having a thickness of 23µm, and cut to form the finished patches.

The amount of the added linoleic acid could be 150g (middle level linoleic acid) or 250g (high level linoleic acid), and all other steps were the same as above described.

The cross section view of the obtained rasagiline transdermal patches with high, middle and low linoleic acid levels is shown in Fig.1.

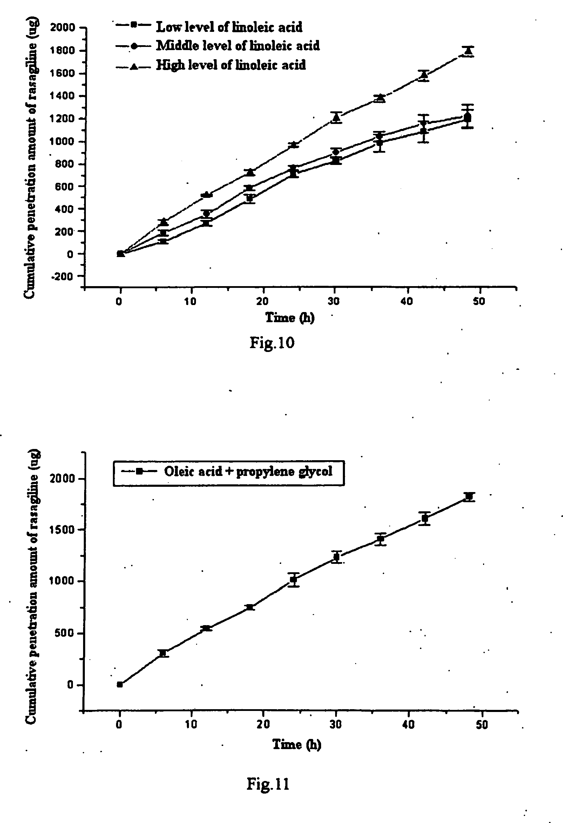

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 4, 5 and 6. Table 4 gives the time-cumulative amount data of rasagiline patch with low level of linoleic acid; Table 5 gives the time-cumulative amount data of rasagiline patch with middle level of linoleic acid; Table 6 gives the time-cumulative amount data of rasagiline patch with high level of linoleic acid. The relation curve diagram of time-cumulative amount of rasagiline is shown in Fig. 10.

It could be seen that the cumulative amounts for all patches were over 1000µg at 48h. The patches with high level of oleic acid had a mean cumulative amount of 1785.25µg at 48h. Thus, this kind of patches can transdermally provides a sufficient amount to achieve systemic effects. As compared to the patches containing oleic acid, no significant difference was observed in the patches containing linoleic acid.

**Table 4. Time-cumulative penetration amount data of rasagiline patches with low level of linoleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 137 | 101 | 114 | 98 | 112.5 | 17.75 |
| 12 | 289 | 299 | 252 | 259 | 274.75 | 22.78 |
| 18 | 532 | 501 | 469 | 449 | 487.75 | 36.45 |
| 24 | 745 | 701 | 688 | 687 | 705.25 | 27.26 |
| 30 | 855 | 832 | 809 | 795 | 822.75 | 26.36 |
| 36 | 918 | 1091 | 980 | 945 | 983.5 | 76.03 |
| 42 | 1190 | 1104 | 978 | 1051 | 1080.75 | 89.29 |
| 48 | 1287 | 1221 | 1094 | 1170 | 1193 | 81.55 |

**Table 5. Time-cumulative penetration amount data of rasagiline patches with middle level of linoleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 187 | 157 | 211 | 193 | 187 | 22.45 |
| 12 | 326 | 319 | 397 | 364 | 351.5 | 36.21 |
| 18 | 584 | 561 | 611 | 587 | 585.75 | 20.45 |
| 24 | 753 | 732 | 774 | 781 | 760 | 22.14 |
| 30 | 917 | 849 | 936 | 881 | 895.75 | 38.62 |
| 36 | 1037 | 993 | 1097 | 1025 | 1038 | 43.50 |
| 42 | 1203 | 1041 | 1210 | 1153 | 1151.75 | 78.07 |
| 48 | 1281 | 1074 | 1273 | 1251 | 1219.75 | 97.99 |

**Table 6. Time-cumulative penetration amount data of rasagiline patches with high level of linoleic acid**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 311 | 255 | 276 | 281 | 280.75 | 23.10 |
| 12 | 532 | 511 | 520 | 517 | 520 | 8.83 |
| 18 | 748 | 704 | 722 | 707 | 720.25 | 20.11 |
| 24 | 984 | 951 | 966 | 951 | 963 | 15.68 |
| 30 | 1267 | 1173 | 1209 | 1159 | 1202 | 48.18 |
| 36 | 1411 | 1345 | 1371 | 1363 | 1372.5 | 27.87 |
| 42 | 1627 | 1573 | 1517 | 1579 | 1574 | 45.03 |
| 48 | 1831 | 1731 | 1788 | 1791 | 1785.25 | 41.14 |

### Example 3: Rasagiline-containing monolayer polyacrylate substrate patches, using oleic acid and propylene glycol as transdermal penetration enhancers.

In chloroform, 50g 50%(w/w) Eudragit E100 solution was added to 200g polyacrylate adhesive fully swollen in aqueous solution, then 250g oleic acid (high oleic acid level) was added, and stirred uniformly.

50g rasagiline was dissolved in 200mL anhydrous ethanol at 50°C~80°C, and then added to the above solution under stirring. 1mol/L NaOH aqueous solution was added slowly under stirring until the PH value was 7.5. After the obtained mixture was stirred uniformly, it was smeared on a medical non-woven fabric by using an appropriate scraper, and the thickness of its wet film was adjusted so that a weight of 60g/m² was obtained after it was dried at 100°C for 60min.

The dried substrate thin film was then covered with a polyester film having a thickness of 23µm, and cut to form the finished patches.

The cross section view of the obtained rasagiline transdermal patches with high level of oleic acid and propylene glycol is shown in Fig.1.

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Table 7. The relation curve diagram of time-cumulative amount of rasagiline is shown in Fig.11.

It could be seen that the cumulative amounts for all patches were over 1000µg at 48h. The patches with high level of oleic acid and propylene glycol had a mean cumulative amount of 1814µg at 48h. Thus, this kind of patches can transdermally provides a sufficient amount to achieve systemic effects. As compared to the patches containing oleic acid at high level, no significant difference was observed in the patches containing the high level of oleic acid and propylene glycol.

**Table 7. Time-cumulative penetration amount data of rasagiline patches with high level of oleic acid and propylene glycol**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 273 | 301 | 292 | 347 | 303.25 | 31.42 |
| 12 | 523 | 532 | 547 | 565 | 541.75 | 18.39 |
| 18 | 727 | 722 | 752 | 773 | 743.50 | 23.64 |
| 24 | 972 | 963 | 999 | 1109 | 1010.75 | 67.26 |
| 30 | 1194 | 1173 | 1244 | 1305 | 1229.00 | 58.77 |
| 36 | 1379 | 1352 | 1403 | 1491 | 1406.25 | 60.22 |
| 42 | 1584 | 1569 | 1572 | 1699 | 1606.00 | 62.34 |
| 48 | 1778 | 1797 | 1809 | 1872 | 1814.00 | 40.72 |

### Example 4: Rasagiline-containing monolayer polyvinylpyrrolidone substrate patches, using isopropyl myristate as transdermal penetration enhancer

20g NaOH and 15g phosphoric acid were added to 500g 25%(w/w) polyvinylpyrrolidone solution, and stirred at 600rpm for 1h. Then, 100g colloid silica was added batchwise, 80g rasagiline and 20g isopropyl myristate (low isopropyl myristate level) were added batchwise, and 1mol/L NaOH aqueous solution was added slowly under stirring until the PH value was 7.5. After the obtained mixture was stirred uniformly for at least 1h, it was smeared on a medical non-woven fabric by using an appropriate scraper, and the thickness of its wet film was adjusted so that a weight of 60g/m² was obtained after it was dried at 80°C for 60min.

The dried substrate thin film was then covered with a polyester film having a thickness of 23µm, and cut to form the finished patches.

The amount of the added isopropyl myristate could be 40g (middle level isopropyl myristate) or 60g (high level isopropyl myristate), and all other steps were the same as above described.

The cross section view of the obtained rasagiline transdermal patches with high, middle and low isopropyl myristate levels is shown in Fig.1.

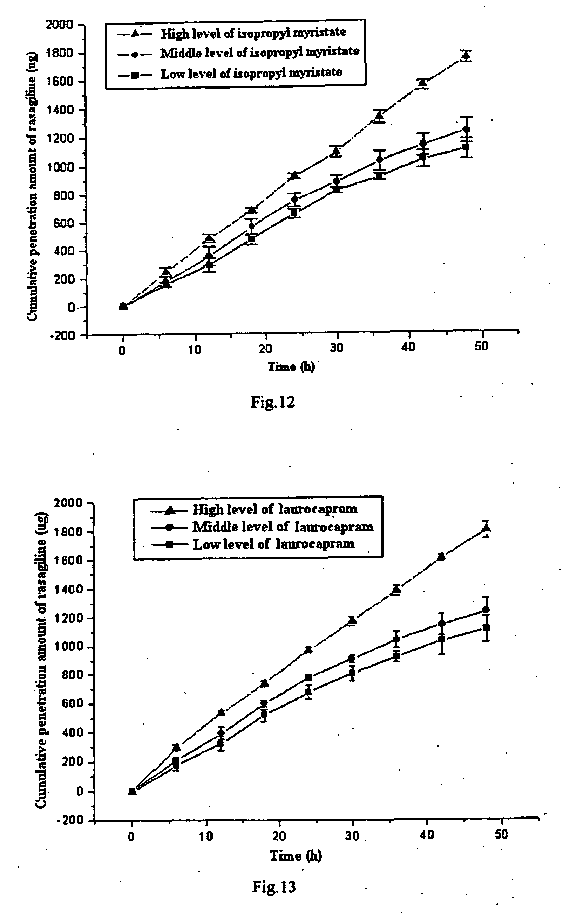

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 8, 9 and 10. Table 8 gives the time-cumulative amount data of rasagiline patch with low level of isopropyl myristate; Table 9 gives the time-cumulative amount data of rasagiline patch with middle level of isopropyl myristate; Table 10 gives the time-cumulative amount data of rasagiline patch with high level of isopropyl myristate. The relation curve diagram of time-cumulative amount of rasagiline is shown in Fig.12.

It could be seen that the cumulative amounts for all patches were over 1000µg at 48h. The patches with high level of isopropyl myristate had a mean cumulative amount of 1741µg at 48h. Thus, this kind of patches can transdermally provides a sufficient amount to achieve systemic effects. The patches with high level of isopropyl myristate had a steeper time-cumulative amount curve, which indicated that their penetration rate was higher. No significant difference was observed between the patches containing low level of isopropyl myristate and the patches containing middle level of isopropyl myristate, which indicated that they were similar in terms of cumulative penetration amount of rasagiline and penetration rate.

**Table 8. Time-cumulative penetration amount data of rasagiline patches with low level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 135 | 156 | 150 | 170 | 152.75 | 14.5 |
| 12 | 230 | 306 | 275 | 346 | 289.25 | 49.04 |
| 18 | 416 | 489 | 490 | 499 | 473.5 | 38.60 |
| 24 | 607 | 673 | 657 | 682 | 654.75 | 33.47 |
| 30 | 799 | 831 | 801 | 838 | 817.25 | 20.14 |
| 36 | 876 | 925 | 920 | 918 | 909.75 | 22.69 |
| 42 | 1041 | 1031 | 962 | 1104 | 1034.5 | 58.14 |
| 48 | 1104 | 1101 | 1014 | 1181 | 1100 | 68.25 |

**Table 9. Time-cumulative penetration amount data of rasagiline patches with middle level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 120 | 190 | 168 | 220 | 174.5 | 42.12 |
| 12 | 280 | 378 | 321 | 432 | 352.75 | 66.38 |
| i8 | 485 | 556 | 579 | 623 | 560.75 | 57.64 |
| 24 | 685 | 778 | 744 | 795 | 750.5 | 48.54 |
| 30 | 825 | 890 | 868 | 926 | 877.25 | 42.25 |
| 36 | 962 | 1014 | 987 | 1115 | 1019.5 | 67.11 |
| 42 | 1130 | 1144 | 1036 | 1211 | 1130.25 | 72.09 |
| 48 | 1210 | 1283 | 1108 | 1295 | 1224 | 85.97 |

**Table 10. Time-cumulative penetration amount data of rasagiline patches with high level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 224 | 258 | 209 | 278 | 242.25 | 31.44 |
| 12 | 467 | 501 | 447 | 503 | 479.5 | 27.25 |
| 18 | 675 | 702 | 658 | 691 | 681.5 | 19.19 |
| 24 | 912 | 931 | 898 | 936 | 919.25 | 17.54 |
| 30 | 1083 | 1117 | 1035 | 1103 | 1084.5 | 35.83 |
| 36 | 1325 | 1365 | 1267 | 1342 | 1324.75 | 41.84 |
| 42 | 1560 | 1580 | 1501 | 1557 | 1549.5 | 33.91 |
| 48 | 1776 | 1747 | 1688 | 1754 | 1741.25 | 37.59 |

### Example 5: Rasagiline-containing silicone polymer substrate patches, using laurocapram as transdermal penetration enhancer

50g rasagiline was dissolved in 200mL anhydrous ethanol at 50°C~80°C, then added to 74% silicone-containing polymer (40g BioPSA 7-4201 + 40g BioPSA 7-4301) heptane solution. After 100g petroleum ether and 50g laurocapram (low laurocapram level) were added, the mixture was stirred at 600rpm for 1h, and 1mol/L NaOH aqueous solution was added slowly under stirring until the PH value was 7.5 to obtain a uniform dispersion. The dispersion was smeared on a medical non-woven fabric by using an appropriate scraper, and the thickness of its wet film was adjusted so that a weight of 60g/m² was obtained after it was dried at 60°C for 60min.

The dried substrate thin film was then covered with a polyester film having a thickness of 23µm, and cut to form the finished patches.

The amount of the added laurocapram could be 150g (middle level laurocapram) or 250g (high level laurocapram ), and all other steps were the same as above described.

The cross section view of the obtained rasagiline transdermal patches with high, middle and low laurocapram levels is shown in Fig.1.

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 11, 12 and 13. Table 11 gives the time-cumulative amount data of rasagiline patch with low level of laurocapram; Table 12 gives the time-cumulative amount data of rasagiline patch with middle level of laurocapram ; Table 13 gives the time-cumulative amount data of rasagiline patch with high level of laurocapram. The relation curve diagram of time-cumulative amount of rasagiline is shown in Fig. 13.

It could be seen that the cumulative amounts for all patches were over 1000µg at 48h. The patches with high level of laurocapram had a mean cumulative amount of 1798.5µg at 48h. Thus, this kind of patches can transdermally provide a sufficient amount to achieve systemic effects. The patches with high level of laurocapram had a steeper time-cumulative amount curve, which indicated that their penetration rate was higher. No significant difference was observed between the patches containing low level of laurocapram and the patches containing middle level of laurocapram, which indicated that they were similar in terms of cumulative penetration amount of rasagiline and penetration rate.

**Table 11. Time-cumulative penetration amount data of rasagiline patches with low level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 125 | 201 | 174 | 190 | 172.5 | 33.55 |
| 12 | 257 | 347 | 310 | 370 | 321 | 49.31 |
| 18 | 466 | 522 | 499 | 568 | 513.75 | 42.85 |
| 24 | 609 | 705 | 653 | 718 | 671.25 | 50.11 |
| 30 | 786 | 811 | 747 | 868 | 803 | 50.71 |
| 36 | 918 | 930 | 866 | 949 | 915.75 | 35.54 |
| 42 | 1070 | 1021 | 893 | 1138 | 1030.5 | 103.46 |
| 48 | 1125 | 1124 | 982 | 1206 | 1109.25 | 93.13 |

**Table 12. Time-cumulative penetration amount data of rasagiline patches with middle level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 212 | 195 | 179 | 226 | 203 | 20.41 |
| 12 | 362 | 394 | 356 | 449 | 390.25 | 42.57 |
| 18 | 580 | 582 | 588 | 627 | 594.25 | 22.10 |
| 24 | 776 | 770 | 753 | 799 | 774.5 | 19.02 |
| 30 | 911 | 892 | 872 | 932 | 901.75 | 25.70 |
| 36 | 1019 | 1011 | 991 | 1121 | 1035.5 | 58.20 |
| 42 | 1191 | 1138 | 1043 | 1204 | 1144 | 73.13 |
| 48 | 1280 | 1257 | 1085 | 1290 | 1228 | 96.33 |

**Table 13. Time-cumulative penetration amount data of rasagiline patches with high level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 312 | 282 | 278 | 303 | 293.75 | 16.38 |
| 12 | 549 | 523 | 514 | 538 | 531 | 15.56 |
| 18 | 761 | 721 | 727 | 725 | 733.5 | 18.50 |
| 24 | 997 | 953 | 963 | 965 | 969.5 | 19.07 |
| 30 | 1196 | 1162 | 1125 | 1197 | 1170 | 34.13 |
| 36 | 1421 | 1361 | 1339 | 1381 | 1375.5 | 34.85 |
| 42 | 1642 | 1603 | 1589 | 1590 | 1606 | 24.83 |
| 48 | 1851 | 1751 | 1743 | 1829 | 1793.5 | 54.54 |

### Example 6: Rasagiline-containing multilayer polyisobutylene substrate patches, using laurocapram as transdermal penetration enhancer

45g rasagiline was dissolved in 200mL anhydrous ethanol at 50°C-80°C, then added to 10%(w/w) polyisobutylene (120g MML-100 + 150g LM-MS) chloroform solution. After 200g petroleum ether and 40g laurocapram (low laurocapram level; while 140g laurocapram was middle level, and 240g laurocapram was high level) were added, the mixture was stirred at 300rpm for 1h, and 1mol/L NaOH aqueous solution was added slowly under stirring until the PH value was 7.5 to obtain a uniform dispersion. The dispersion was smeared on a medical non-woven fabric by using an appropriate scraper, the thickness of its wet film was adjusted, and the film was dried at 60°C for 60min for standby.

5g rasagiline was dissolved in 200mL anhydrous ethanol at 50°C~80°C, then added to 10%(w/w) polyisobutylene (12g MML-100 + 15g LM-MS) chloroform solution. After 30g petroleum ether and 10g laurocapram were added, the mixture was stirred at 600rpm for 1h to obtain a uniform dispersion. The dispersion was smeared on the above film using an appropriate scraper, the thickness of this wet film was adjusted, so that the weight was 60g/m² after the film was dried at 60°C for 60min.

The dried substrate thin film was then covered with a polyester film having a thickness of 23µm, and cut to form the finished patches.

The cross section view of the obtained rasagiline transdermal patches with high, middle and low laurocapram levels is shown in Fig.2.

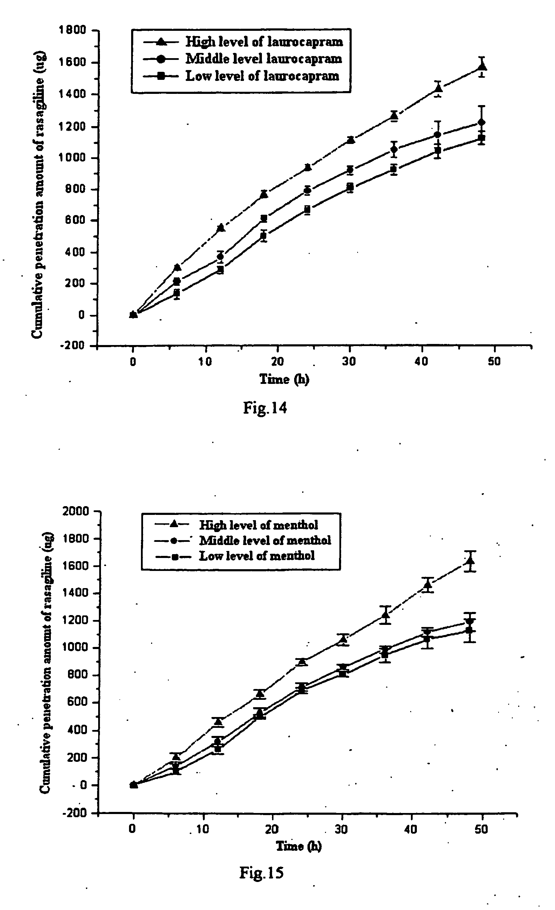

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 14, 15 and 16. Table 14 gives the time-cumulative amount data of rasagiline patch with low level of laurocapram ; Table 15 gives the time-cumulative amount data of rasagiline patch with middle level of laurocapram ; Table 16 gives the time-cumulative amount data of rasagiline patch with high level of laurocapram . The relation curve diagram of time-cumulative amount of rasagiline is shown in Fig.14.

It could be seen that the cumulative amounts for all patches were over 1000µg at 48h. The patches with high level of laurocapram had a mean cumulative amount of 1818.75µg at 48h. Thus, this kind of patches can transdermally provide a sufficient amount to achieve systemic effects. The patches with high level of laurocapram had a steeper time-cumulative amount curve, but the curve became plain after 18h indicating the reduction of rasagiline penetration rate, which suggested that the rasagiline penetration rates of the two polyisobutylene layers were different. No significant difference was observed between the patches containing low level of laurocapram and the patches containing middle level of laurocapram in term of the time-cumulative amount of rasagiline, which indicated that they were similar in terms of cumulative penetration amount of rasagiline and penetration rate.

**Table 14. Time-cumulative penetration amount data of rasagiline patches with low level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 138 | 119 | 177 | 106 | 135 | 30.93 |
| 12 | 271 | 271 | 315 | 298 | 288.75 | 21.64 |
| 18 | 488 | 475 | 559 | 490 | 503 | 37.92 |
| 24 | 642 | 647 | 703 | 675 | 666.75 | 28.19 |
| 30 | 796 | 773 | 842 | 807 | 804.5 | 28.73 |
| 36 | 905 | 905 | 973 | 910 | 923.25 | 33.25 |
| 42 | 1086 | 996 | 1006 | 1075 | 1040.75 | 46.30 |
| 48 | 1101 | 1172 | 1083 | 1154 | 1127.5 | 42.29 |

**Table 15. Time-cumulative penetration amount data of rasagiline patches with middle level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 227 | 201 | 190 | 237 | 213.75 | 21.93 |
| 12 | 351 | 362 | 335 | 420 | 367 | 37.03 |
| 18 | 610 | 596 | 601 | 641 | 612 | 24.18 |
| 24 | 796 | 769 | 758 | 821 | 786 | 28.27 |
| 30 | 941 | 907 | 882 | 944 | 918.5 | 29.56 |
| 36 | 1047 | 1025 | 1009 | 1125 | 1051.5 | 51.42 |
| 42 | 1223 | 1121 | 1042 | 1208 | 1148.5 | 84.04 |
| 48 | 1300 | 1241 | 1073 | 1285 | 1224.75 | 104.22 |

**Table 16. Time-cumulative penetration amount data of rasagiline patches with high level of laurocapram**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 318 | 289 | 294 | 307 | 302 | 13.09 |
| 12 | 564 | 537 | 555 | 546 | 550.5 | 11.62 |
| 18 | 794 | 765 | 762 | 739 | 765 | 22.55 |
| 24 | 963 | 928 | 940 | 917 | 937 | 19.71 |
| 30 | 1139 | 1116 | 1103 | 1092 | 1112.5 | 20.21 |
| 36 | 1305 | 1250 | 1268 | 1233 | 1264 | 30.84 |
| 42 | 1479 | 1457 | 1433 | 1369 | 1434.5 | 47.54 |
| 48 | 1645 | 1591 | 1544 | 1501 | 1570.25 | 61.92 |

### Example 7: Rasagiline-containing multilayer ethylene-vinyl acetate copolymer substrate patches, using menthol as transdermal penetration enhancer

50g rasagiline was added to 25% (w/w) ethylene-vinyl acetate copolymer (120g Celgard 2400) chloroform solution. After 50g menthol (low menthol level; while 150g menthol was middle level, and 250g menthol was high level) was added, the mixture was stirred at 600rpm for 1h to obtain a uniform dispersion, and 1mol/L NaOH aqueous solution was added slowly under stirring until the PH value was 7.5. After agitated uniformly, the dispersion was then smeared on a medical non-woven fabric by using an appropriate scraper to form a round shape having area of about 30cm², the thickness of its wet film was adjusted, and the film was dried at 60°C for 60min for standby.

To 74% silicone-containing polymer (40g BioPSA 7-4201 + 40g BioPSA 7-4301) heptane solution, 100g petroleum ether was added, and the mixture was stirred at 600rpm for 1h to obtain a uniform dispersion. The dispersion was smeared around the above round shape using an appropriate scraper, the thickness of this wet film was adjusted, so that the weight was 60g/m² after the film was dried at 60°C for 60min.

The dried substrate thin film was then covered with a polyester film having a thickness of 23µm, and cut to form the finished patches.

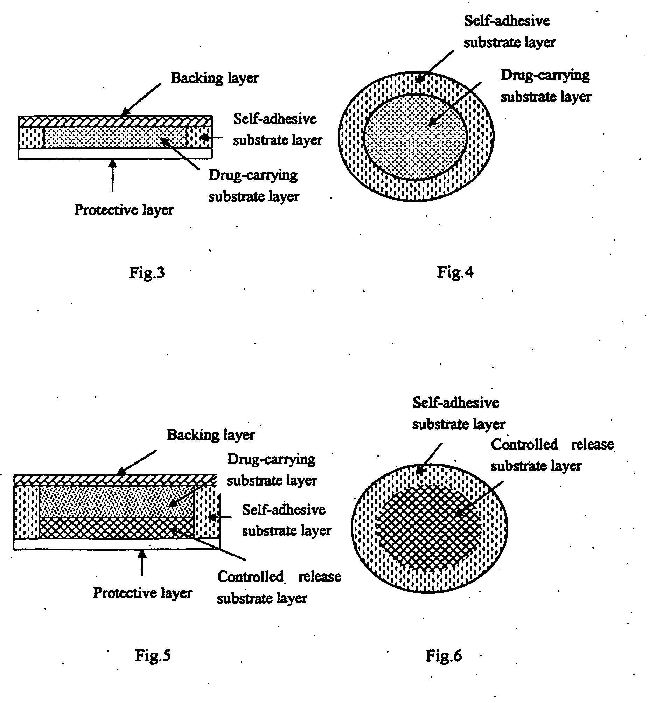

The cross section view of the obtained rasagiline transdermal patches with high, middle and low menthol levels is shown in Fig.3, and their plane view is shown in Fig.4.

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 17, 18 and 19. Table 17 gives the time-cumulative amount data of rasagiline patch with low level of menthol; Table 18 gives the time-cumulative amount data of rasagiline patch with middle level of menthol; Table 19 gives the time-cumulative amount data of rasagiline patch with high level of menthol. The relation curve diagram of time-cumulative amount of rasagiline is shown in Fig.15.

It could be seen that the patches with high level of menthol had a mean cumulative amount of 1633.25µg at 48h, and the relation curve of time-cumulative amount of rasagiline was substantively present in a straight line. The relation curves of time-cumulative amount of rasagiline between the patches containing low level of menthol and that containing middle level of menthol are very similar, which indicated that they were similar in terms of cumulative penetration amount of rasagiline and penetration rate.

**Table 17. Time-cumulative penetration amount data of rasagiline patches with low level of menthol**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 102 | 97 | 85 | 124 | 102 | 16.31 |
| 12 | 245 | 274 | 231 | 311 | 265.25 | 35.37 |
| 18 | 501 | 478 | 502 | 519 | 500 | 16.83 |
| 24 | 664 | 698 | 666 | 702 | 682.5 | 20.29 |
| 30 | 801 | 785 | 798 | 833 | 804.25 | 20.39 |
| 36 | 944 | 907 | 909 | 1012 | 943 | 49.04 |
| 42 | 1109 | 1054 | 965 | 1109 | 1059.25 | 67.97 |
| 48 | 1171 | 1154 | 1001 | 1177 | 1125.75 | 83.74 |

**Table 18. Time-cumulative penetration amount data of rasagiline patches with middle level of menthol**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 138 | 159 | 161 | 122 | 145 | 18.53 |
| 12 | 266 | 351 | 322 | 343 | 320.5 | 38.34 |
| 18 | 487 | 539 | 563 | 522 | 527.75 | 31.95 |
| 24 | 671 | 728 | 736 | 698 | 708.25 | 29.74 |
| 30 | 851 | 882 | 852 | 841 | 856.5 | 17.71 |
| 36 | 969 | 997 | 1018 | 961 | 986.25 | 26.20 |
| 42 | 1137 | 1118 | 1067 | 1132 | 1113.5 | 32.03 |
| 48 | 1208 | 1251 | 1094 | 1208 | 1190.25 | 67.29 |

**Table 19. Time-cumulative penetration amount data of rasagiline patches with high level of menthol**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 235 | 256 | 213 | 266 | 242.5 | 23.530 |
| 12 | 475 | 497 | 452 | 495 | 479.75 | 20.998 |
| 18 | 686 | 707 | 668 | 677 | 684.5 | 16.703 |
| 24 | 834 | 848 | 786 | 872 | 835 | 36.240 |
| 30 | 991 | 1025 | 926 | 1008 | 987.5 | 43.286 |
| 36 | 1156 | 1180 | 1083 | 1173 | 1148 | 44.490 |
| 42 | 1425 | 1302 | 1234 | 1281 | 1310.5 | 81.456 |
| 48 | 1535 | 1496 | 1421 | 1478 | 1482.5 | 47.403 |

### Example 8: Rasagiline-containing patches with carbopol as substrate, ethylene-vinyl acetate copolymer as controlled release membrane, and isopropyl myristate as transdermal penetration enhancer

2g carbopol (carbopol 940) was spread to 200g 70% (v/v) ethanol-pure water solution, and fully swollen by heating and fluxing at 80°C for 4h, then 45g rasagiline, 40g isopropyl myristate (low isopropyl myristate level; while 140g isopropyl myristate was middle level, and 240g isopropyl myristate was high level) and 40g glycerol were added in order, the mixture was stirred at 300rpm for 1h to obtain an uniform dispersion, and 0.5mol/L triethanolamine solution was added slowly under 30rpm stirring until the PH value was 8.5, so that it became a gel with the maximum viscosity. The dispersion was then smeared around the round shape using an appropriate scraper, and the thickness of its wet film was adjusted.

5g rasagiline was dissolved in 200mL anhydrous ethanol at 50°C~80°C, then added to 15% (w/w) ethylene-vinyl acetate copolymer (15g Celgard 2400) chloroform solution. After 30g petroleum ether and 10g isopropyl myristate were added, the mixture was stirred at 300rpm for 1h to obtain a uniform dispersion. The dispersion was smeared on the above film, the thickness of this wet film was adjusted, so that the weight was 60g/m² after the film was dried at 60°C for 60min.

To 74% silicone-containing polymer (40g BioPSA 7-4201 + 40g BioPSA 7-4301) heptane solution, 100g petroleum ether was added, and the mixture was stirred at 300rpm for 1h to obtain a uniform dispersion. The dispersion was smeared around the above round shape using an appropriate scraper, the thickness of this wet film was adjusted, so that the weight was 60g/m² after the film was dried at 60°C for 60min.

The dried substrate thin film was then covered with a polyester film having a thickness of 23µm, and cut to form the finished patches.

The cross section view of the obtained rasagiline transdermal patches with high, middle and low isopropyl myristate levels is shown in Fig.5, and their plane view is shown in Fig. 6.

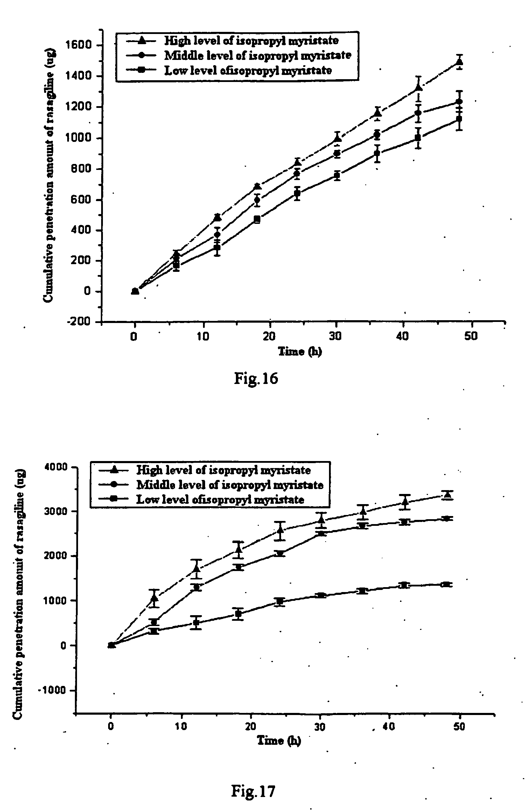

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 20, 21 and 22. Table 20 gives the time-cumulative amount data of rasagiline patch with low level of isopropyl myristate; Table 21 gives the time-cumulative amount data of rasagiline patch with middle level of isopropyl myristate; Table 22 gives the time-cumulative amount data of rasagiline patch with high level of isopropyl myristate. The relation curve diagram of time-cumulative amount of rasagiline is shown in Fig. 16.

It could be seen that the cumulative amounts for all patches were over 1000µg at 48h. The rasagiline patches with high level of isopropyl myristate had a mean cumulative amount of 1635.25µg at 48h, and the rasagiline penetration rate became small after 18h, which indicated that ethylene-vinyl acetate copolymer controlled release membrane exhibited controlled release effects. The patches containing low level of isopropyl myristate are different from the patches containing middle level of isopropyl myristate in terms of cumulative penetration amount of rasagiline and penetration rate, but the difference is smaller than the difference in comparison with the patches containing high level of isopropyl myristate.

**Table 20. Time-cumulative penetration amount data of rasagiline patches with low level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 137 | 169 | 144 | 203 | 163.25 | 29.85 |
| 12 | 224 | 301 | 256 | 345 | 281.5 | 52.82 |
| 18 | 436 | 477 | 468 | 487 | 467 | 22.08 |
| 24 | 576 | 650 | 676 | 650 | 638 | 43.11 |
| 30 | 712 | 765 | 746 | 780 | 750.75 | 29.34 |
| 36 | 855 | 882 | 864 | 975 | 894 | 55.15 |
| 42 | 999 | 1002 | 912 | 1064 | 994.25 | 62.48 |
| 48 | 1110 | 1155 | 1015 | 1175 | 1113.75 | 71.22 |

**Table 21. Time-cumulative penetration amount data of rasagiline patches with middle level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 184 | 240 | 211 | 202 | 209.25 | 23.37 |
| 12 | 303 | 401 | 358 | 405 | 366.75 | 47.53 |
| 18 | 531 | 623 | 616 | 599 | 592.25 | 42.06 |
| 24 | 721 | 811 | 777 | 759 | 767 | 37.49 |
| 30 | 855 | 912 | 905 | 896 | 892 | 25.52 |
| 36 | 973 | 1031 | 1031 | 1018 | 1013.25 | 27.52 |
| 42 | 1165 | 1155 | 1075 | 1212 | 1151.75 | 56.88 |
| 48 | 1233 | 1275 | 1128 | 1265 | 1225.25 | 67.26 |

**Table 22. Time-cumulative penetration amount data of rasagiline patches with high level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6 | 235 | 256 | 213 | 266 | 242.5 | 23.530 |
| 12 | 475 | 497 | 452 | 495 | 479.75 | 20.998 |
| 18 | 686 | 707 | 668 | 677 | 684.5 | 16.703 |
| 24 | 834 | 848 | 786 | 872 | 835 | 36.240 |
| 30 | 991 | 1025 | 926 | 1008 | 987.5 | 43.286 |
| 36 | 1156 | 1180 | 1083 | 1173 | 1148 | 44.490 |
| 42 | 1425 | 1302 | 1234 | 1281 | 1310.5 | 81.456 |
| 48 | 1535 | 1496 | 1421 | 1478 | 1482.5 | 47.403 |

Thus, the patches according to the present invention having an area of about 5~10cm² were able to transdermally provide a therapeutic dose needed for 2~3 days treatment, and the penetration rate of rasagiline could be controlled by using multilayer patches. Since the raw materials for preparing the patches are widely and readily available, and rasagiline could be transferred through the whole surface of the substrate for transdermal absorption, the patches of the present invention could be prepared simply with widely available raw materials, have smooth release profile and a prolonged period for controlled release, and may be more effectively absorbed through skin in comparison with the system as described in US2004013620.

### Example 9: Rasagiline-containing patches with polyvinyl alcohol copolymer as substrate and isopropyl myristate as transdermal penetration enhancer

50g polyvinyl alcohol copolymer (50g PVA124) was spread to 450g pure water solution, and fully swollen by heating and fluxing at 80°C for 4h, then 25g rasagiline was added. After 5g isopropyl myristate (low isopropyl myristate level; while 10g isopropyl myristate was middle level, and 20g isopropyl myristate was high level) was added, the mixture was stirred at 600rpm for 1h to obtain a uniform dispersion. After agitated uniformly, the dispersion was then smeared on a medical non-woven fabric using an appropriate scraper to form a round shape having an area about 30cm², the thickness of its wet film was adjusted, and the film was dried at 60°C for 70min for standby.

To 74% silicone-containing polymer (40g BioPSA 7-4201 + 40g BioPSA 7-4301) heptane solution, 100g petroleum ether was added, and the mixture was stirred at 600rpm for 1h to obtain a uniform dispersion. The dispersion was smeared around the above round shape using an appropriate scraper, the thickness of this wet film was adjusted, and the film was dried at 60°C.

The dried substrate thin film was then covered with a polyester film having a thickness of 23µm, and cut to form the finished patches.

The cross section view of the obtained rasagiline transdermal patches with high, middle and low isopropyl myristate levels is shown in Fig.3 and their plane view is shown in Fig.4.

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Tables 23, 24 and 25. Table 23 gives the time-cumulative amount data of rasagiline patch with low level of isopropyl myristate; Table 24 gives the time-cumulative amount data of rasagiline patch with middle level of isopropyl myristate; Table 25 gives the time-cumulative amount data of rasagiline patch with high level of isopropyl myristate. The relation curve diagram of time-cumulative amount of rasagiline is shown in Fig. 17.

It could be seen that the patches with high level of isopropyl myristate had a mean cumulative amount of 3362.25µg at 48h, and the relation curve of time-cumulative amount of rasagiline was substantively present in a straight line. The relation curves of time-cumulative amount for rasagiline patches containing high and middle levels of isopropyl myristate were relatively close, which indicated the patches containing the two levels of isopropyl myristate had almost the same cumulative penetration amount of rasagiline and penetration rate. The patches of Example 9 had a cumulative penetration amount significantly higher than those of Examples 1~8, which indicated that polyvinyl alcohol copolymer absorbs moisture to form a saturated solution of rasagiline and greatly enhances the transdermal effects of rasagiline.

**Table 23. Time-cumulative penetration amount data of rasagiline patches with low level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6h | 350 | 399 | 247 | 293 | 322.25 | 66.28 |
| 12h | 588 | 675 | 362 | 407 | 508 | 148.11 |
| 18h | 815 | 823 | 577 | 601 | 704 | 133.19 |
| 24h | 935 | 1097 | 893 | 990 | 978.75 | 88.27 |
| 30h | 1121 | 1190 | 1096 | 1103 | 1127.5 | 42.98 |
| 36h | 1233 | 1287 | 1160 | 1207 | 1221.75 | 52.96 |
| 42h | 1338 | 1392 | 1272 | 1364 | 1341.5 | 51.31 |
| 48h | 1379 | 1355 | 1315 | 1380 | 1357.25 | 30.45 |

**Table 24. Time-cumulative penetration amount data of rasagiline patches with middle level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6h | 558 | 432 | 509 | 590 | 522.25 | 68.77 |
| 12h | 1322 | 1201 | 1298 | 1379 | 1300 | 74.23 |
| 18h | 1788 | 1679 | 1702 | 1795 | 1741 | 59.13 |
| 24h | 2111 | 1998 | 2005 | 2113 | 2056.75 | 63.87 |
| 30h | 2521 | 2437 | 2488 | 2533 | 2494.75 | 42.94 |
| 36h | 2703 | 2577 | 2689 | 2687 | 2664 | 58.44 |
| 42h | 2789 | 2689 | 2715 | 2832 | 2756.25 | 65.92 |
| 48h | 2850 | 2803 | 2783 | 2860 | 2824 | 36.94 |

**Table 25. Time-cumulative penetration amount data of rasagiline patches with high level of isopropyl myristate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0 |
| 6h | 1103 | 1001 | 1305 | 807 | 1054 | 207.54 |
| 12h | 1725 | 1639 | 1962 | 1462 | 1697 | 207.84 |
| 18h | 2177 | 2081 | 2344 | 1903 | 2126.25 | 184.28 |
| 24h | 2604 | 2498 | 2811 | 2315 | 2557 | 207.18 |
| 30h | 2854 | 2743 | 2987 | 2583 | 2791.75 | 171.22 |
| 36h | 2999 | 2932 | 3198 | 2789 | 2979.5 | 169.97 |
| 42h | 3218 | 3175 | 3401 | 3001 | 3198.75 | 164.26 |
| 48h | 3357 | 3302 | 3491 | 3299 | 3362.25 | 89.88 |

Thus, the patches according to the present invention having an area of about 5~10cm² were able to transdermally provide a therapeutic dose needed for 2~3 days treatment, and the penetration rate of rasagiline could be controlled by using multilayer patches. Since the raw materials for preparing the patches are widely and readily available, and rasagiline could be transferred through the whole surface of the substrate for transdermal absorption, the patches of the present invention could be prepared simply with widely available raw materials, have smooth release profile and a prolonged period for controlled release, and may be more effectively absorbed through skin in comparison with the system as described in US2004013620.

### Example 10: Rasagiline-containing multilayer patches with polyvinyl alcohol copolymer as substrate and isopropyl myristate as transdermal penetration enhancer

50g polyvinyl alcohol copolymer (50g PVA124) was spread to 450g pure water solution, and fully swollen by heating and fluxing at 80°C for 4h, then 100g triethanolamine was added thereto. After 10g isopropyl myristate was added, the mixture was stirred at 600rpm for 1h to obtain a uniform dispersion. After agitated uniformly, the dispersion was then smeared on a medical non-woven fabric using an appropriate scraper to form a round shape having an area about 30cm², the thickness of its wet film was adjusted, and the film was dried at 70°C for 60min for standby.

50g polyvinyl alcohol copolymer (50g PVA124) was spread to 450g pure water solution, and fully swollen by heating and fluxing at 80°C for 4h, then 25g rasagiline was added thereto. After 10g isopropyl myristate was added, the mixture was stirred at 600rpm for 1h to obtain a uniform dispersion. After agitated uniformly, the dispersion was then smeared on the above film using an appropriate scraper to form a round shape having an area about 30cm² the thickness of its wet film was adjusted, and the film was dried at 70°C for 60min for standby.

To 74% silicone-containing polymer (40g BioPSA 7-4201 + 40g BioPSA 7-4301) heptane solution, 100g petroleum ether was added, and the mixture was stirred at 600rpm for 1h to obtain a uniform dispersion. The dispersion was smeared around the above round shape using an appropriate scraper, the thickness of this wet film was adjusted, and the film was dried at 60°C.

The dried substrate thin film was then covered with a polyester film having a thickness of 23µm, and cut to form the finished patches.

The cross section view of the obtained rasagiline transdermal patches is shown in Fig.7 and their plane view is shown in Fig. 8.

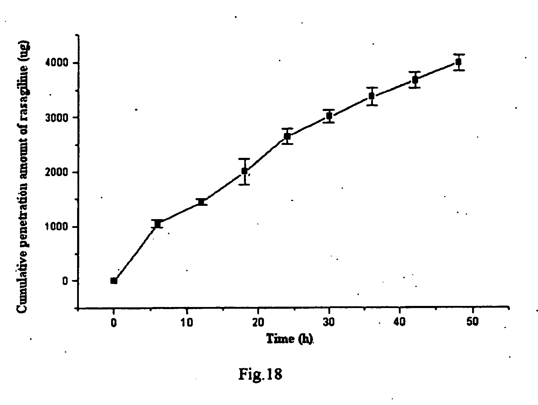

The penetration properties of the finished patches were studied by using Franz cell and skin of abdomen of hairless mice. Rasagiline contents were measured by using high performance liquid chromatograph at different time points, the cumulative penetration amount of rasagiline was calculated accordingly, and the results were shown in Table 26. The relation curve diagram of time-cumulative amount of rasagiline is shown in Fig. 18.

It could be seen that the rasagiline multilayer patches of Example 10 had a mean cumulative amount of 3970.5µg at 48h, and the relation curve of time-cumulative amount of rasagiline was substantively present in a straight line. The patches of Example 10 had a cumulative penetration amount significantly higher than those of Examples 1~8. As compared to Example 9, the patches of Example 10 with isopropyl myristate at middle level still had a higher cumulative penetration amount than the patches of Example 9 with isopropyl myristate at high level, which indicated that polyvinyl alcohol copolymer absorbs moisture to form a saturated solution of rasagiline and the penetration of triethanolamine of the regulator substrate layer causes dissociation of rasagiline, thereby further enhancing the transdermal effects of rasagiline.

**Table 26. Time-cumulative penetration amount data of rasagiline multilayer patches with polyvinyl alcohol copolymer as substrate**

| Time | Group 1 | Group 2 | Group 3 | Group 4 | Mean | SD |
|---|---|---|---|---|---|---|
| (h) | (µg) | (µg) | (µg) | (µg) | (µg) | (µg) |
| 0 | 0 | 0 | 0 | 0 | 0 | 0.00 |
| 6h | 1058 | 977 | 1133 | 1007 | 1043.75 | 68.25 |
| 12h | 1524 | 1457 | 1431 | 1387 | 1449.75 | 57.31 |
| 18h | 2283 | 2098 | 1831 | 1795 | 2001.75 | 231.13 |
| 24h | 2804 | 2681 | 2534 | 2508 | 2631.75 | 137.80 |
| 30h | 3134 | 3029 | 2891 | 2903 | 2989.25 | 114.93 |
| 36h | 3497 | 3465 | 3157 | 3289 | 3352 | 158.94 |
| 42h | 3705 | 3811 | 3481 | 3605 | 3650.5 | 140.87 |
| 48h | 4033 | 4132 | 3795 | 3922 | 3970.5 | 145.08 |

Thus, the patches according to the present invention having an area of about 5~10cm² were able to transdermally provide a therapeutic dose needed for 2~3 days treatment, and the penetration rate of rasagiline could be controlled by using multilayer patches. Since the raw materials for preparing the patches are widely and readily available, and rasagiline could be transferred through the whole surface of the substrate for transdermal absorption, the patches of the present invention could be prepared simply with widely available raw materials, have smooth release profile and a prolonged period for controlled release, and may be more effectively absorbed through skin in comparison with the system as described in US2004013620.

## Claims

1. A rasagiline transdermal patch for treatment or prophylaxis of nervous system diseases, wherein the patch comprises an inert backing layer chemically inert to substrate ingredients, a substrate layer comprising rasagiline or a pharmaceutically acceptable salt thereof, and a protective layer to be peeled off before use, wherein the substrate layer is a drug-carrying reservoir comprising an organic polymer material and an inorganic or organic material as regulator, the reservoir comprises rasagiline, and the substrate layer further comprises one or more substances for enhancing the transdermal absorption of rasagiline wherein the rasagiline or pharmaceutically acceptable salt thereof in the substrate layer is present in the form of free base.

2. The rasagiline transdermal patch according to claim 1, wherein the substrate layer further comprises a controlled release substrate layer and/or an adhesive layer.

3. The rasagiline transdermal patch according to claim 1, wherein the rasagiline or pharmaceutically acceptable salt thereof in the substrate layer has an effective amount of 0.01mg/cm²~50mg/cm².

4. The rasagiline transdermal patch according to any one of claims 1 to 3, wherein the pharmaceutically acceptable salt of rasagiline comprises hydrochloride, mesylate, esylate, or sulfate.

5. The rasagiline transdermal patch according to claim 4, wherein the pharmaceutically acceptable salt of rasagiline is mesylate.

6. The rasagiline transdermal patch according to claim 1, wherein the organic polymer material comprises at least one type of the following organic polymer materials:
polyacrylates and derivatives thereof, polyvinylpyrrolidone and derivatives thereof, silicone polymers and derivatives thereof, polyvinyl alcohol and derivatives thereof, polyisobutylene and derivatives thereof, ethylene-vinyl acetate copolymers and derivatives thereof, and carbopol and derivatives thereof.

7. The rasagiline transdermal patch according to claim 1, wherein the inorganic or organic material as regulator comprises one or more of the following substances: water, sodium hydroxide, sodium phosphate, sodium hydrogen phosphate, sodium dihydrogen phosphate, sodium carbonate, sodium hydrogen carbonate, sodium chloride, sodium sulfate, sodium hydrogen sulfate, potassium hydroxide, potassium phosphate, potassium hydrogen phosphate, potassium dihydrogen phosphate, potassium carbonate, potassium hydrogen carbonate, potassium chloride, potassium sulfate, potassium hydrogen sulfate, calcium hydroxide, calcium phosphate, calcium hydrogen phosphate, calcium carbonate, calcium chloride, calcium sulfate, phosphoric acid, hydrochloric acid, sulfuric acid, silicon dioxide, silicic acid, sodium metasilicate, potassium metasilicate, sodium trisilicate, potassium trisilicate, titanium dioxide, ethanediamine, triethylamine, triethanolamine, methanol, ethanol, propanol, propylene glycol, glycerol, butanol, chloroform, dichloromethane, tetrachloromethane, urea, petroleum ether, polyvidone, polyethylene glycol, long-chain fatty alcohols, oils and lipids.

8. The rasagiline transdermal patch according to claim 1, wherein the substance for enhancing the transdermal absorption of rasagiline comprises at least one of the following substances: ethanol, propylene glycol, oleic acid, ocenol, linoleic acid, menthol, laurinol, lauric acid, isopropyl myristate, azacycloheptan-2-ones and terpenes.

9. The rasagiline transdermal patch according to claim 1, wherein the inorganic or organic material comprises an alkaline substance.

10. The rasagiline transdermal patch according to claim 9, wherein the alkaline substance is sodium hydroxide or triethanolamine.

11. A method for preparing a rasagiline transdermal patch, wherein the method comprises the following steps:
a) Blending an effective amount of rasagiline or a pharmaceutically acceptable salt thereof into an organic polymer material pre-heated to 50°C~200°C;
b) Adding a regulator and a substance for enhancing transdermal absorption, mixing uniformly to obtain a mixture substrate so that the rasagiline or a pharmaceutically acceptable salt thereof in the substrate is present in the form of free base; and
c) Smearing the mixture substrate on an inert backing layer to form a uniform thin film, and sticking thereon a protective layer to obtain the patch.

12. A method for preparing a rasagiline transdermal patch, wherein the method comprises the following steps:
a) Adding an effective amount of rasagiline or a pharmaceutically acceptable salt thereof to a solvent with a desired volatility, adding thereto and fully swelling an organic polymer material;
b) Adding thereto simultaneously or in order a regulator and a substance for enhancing transdermal absorption to obtain a mixture substrate so that the rasagiline or pharmaceutically acceptable salt thereof is present in the form of free base; and
c) Smearing the mixture substrate on an inert backing layer to form a uniform thin film, and sticking thereon a protective layer to obtain the patch.

13. The method for preparing a rasagiline transdermal patch according to claim 11 or 12, wherein the step c) further comprises repeating the smearing by any way to obtain a multilayer thin film substrate.

14. The method for preparing a rasagiline transdermal patch according to claim 11 or 12, wherein the step c) may further comprise an adhesive layer free of rasagiline as the outmost layer.

15. The rasagiline transdermal patch for use in the treatment or prophylaxis of a nervous system disease according to claim 1, wherein the nervous system disease comprises Parkinson's disease, Alzheimer's disease, depression, hyperactive child syndrome, restless leg syndrome, multiple sclerosis and abstinence syndrome.

## Patentansprüche

1. Ein transdermales Pflaster mit Rasagilin zur Behandlung oder Prophylaxe von Erkrankungen des Nervensystems, wobei das Pflaster eine inerte Trägerschicht umfasst, die chemisch inert gegenüber Substratinhaltsstoffen ist, eine Substratschicht mit Rasagilin oder einem pharmazeutisch verträglichen Salz davon, und eine Schutzschicht umfasst, die vor dem Gebrauch abgezogen wird, wobei die Substratschicht ein Wirkstoff-Reservoir ist, dass ein organisches Polymermaterial und ein anorganisches oder organisches Material als Regulator umfasst, das Reservoir Rasagilin beinhaltet, und die Substratschicht ferner einen oder mehrere Stoffe zur Erhöhung der transdermalen Absorption von Rasagilin umfasst, wobei das Rasagilin oder das pharmazeutisch verträgliche Salz davon in der Substratschicht in Form einer freien Base vorliegt.

2. Das transdermale Pflaster mit Rasagilin nach Anspruch 1, wobei die Substratschicht ferner eine Substratschicht mit kontrollierter Freisetzung und / oder eine Haftschicht umfasst.

3. Das transdermale Pflaster mit Rasagilin nach Anspruch 1, wobei das Rasagilin oder das pharmazeutisch verträgliche Salz davon in der Substratschicht eine wirksame Menge von 0,01 mg/cm² ~ 50 mg/cm² hat.

4. Das transdermale Pflaster mit Rasagilin nach einem der Ansprüche 1 bis 3, wobei das pharmazeutisch verträgliche Salz von Rasagilin Hydrochlorid, Mesylat, Esylat oder Sulfat umfasst.

5. Das transdermale Pflaster mit Rasagilin nach Anspruch 4, wobei das pharmazeutisch verträgliche Salz von Rasagilin Mesylat ist.

6. Das transdermale Pflaster mit Rasagilin nach Anspruch 1, wobei das organische Polymermaterial mindestens eine Art der folgenden organischen Polymermaterialien umfasst: Polyacrylate und Derivate davon, Polyvinylpyrrolidon und Derivate davon, Silikonpolymere und Derivate davon, Polyvinylalkohol und Derivate davon, Polyisobutylen und Derivate davon, Ethylen-Vinylacetat-Copolymere und Derivate davon, und Carbopol und Derivate davon.

7. Das transdermale Pflaster mit Rasagilin nach Anspruch 1, wobei das anorganische oder organische Material als Regulator einen oder mehrere der folgenden Stoffe umfasst:
Wasser, Natriumhydroxid, Natriumphosphat, Natriumhydrogenphosphat, Natriumdihydrogenphosphat, Natriumcarbonat, Natriumhydrogencarbonat, Natriumchlorid, Natriumsulfat, Natriumhydrogensulfat, Kaliumhydroxid, Kaliumphosphat, Kaliumhydrogenphosphat, Kaliumdihydrogenphosphat, Kaliumcarbonat, Kaliumhydrogencarbonat, Kaliumchlorid, Kaliumsulfat, Kaliumhydrogensulfat, Calciumhydroxid, Calciumphosphat, Calciumhydrogenphosphat, Calciumcarbonat, Calciumchlorid, Calciumsulfat, Phosphorsäure, Salzsäure, Schwefelsäure, Siliziumdioxid, Kieselsäure, Natriummetasilikat, Kaliummetasilikat, Natriumtrisilikat, Kaliumtrisilikat, Titandioxid, Ethandiamin, Triethylamin, Triethanolamin, Methanol, Ethanol, Propanol, Propylenglykol, Glycerin, Butanol, Chloroform, Dichlormethan, Tetrachlorkohlenstoff, Harnstoff, Petrolether, Polyvidon, Polyethylenglykol, langkettige Fettalkohole, Öle und Lipide.

8. Das transdermale Pflaster mit Rasagilin nach Anspruch 1, wobei der Stoff zur Erhöhung der transdermalen Absorption von Rasagilin mindestens einen der folgenden Stoffe umfasst: Ethanol, Propylenglykol, Ölsäure, Ocenol, Linolsäure, Menthol, Laurinol, Laurinsäure, Isopropylmyristat, Azacycloheptan-2-one und Terpene.

9. Das transdermale Pflaster mit Rasagilin nach Anspruch 1, wobei das anorganische oder organische Material einen alkalischen Stoff umfasst.

10. Das transdermale Pflaster mit Rasagilin nach Anspruch 9, wobei der alkalische Stoff Natriumhydroxid oder Triethanolamin ist.

11. Ein Verfahren zur Herstellung eines transdermalen Pflasters mit Rasagilin, wobei das Verfahren die folgenden Schritte umfasst:
a) Mischen einer wirksamen Menge von Rasagilin oder eines pharmazeutisch verträglichen Salzes davon in ein auf 50°C - 200°C vorgewärmtes organisches Polymermaterial;
b) Hinzufügen eines Regulators und eines Stoffes zur Erhöhung der transdermalen Absorption, einheitliches Vermischen um eine Substratmischung zu erhalten, so dass das Rasagilin oder ein pharmazeutisch verträgliches Salz davon in dem Substrat in Form einer freien Base vorliegt; und
c) Verschmieren der Substratmischung auf einer inerten Trägerschicht unter Bildung eines gleichmäßigen dünnen Films, und Aufkleben einer Schutzschicht um das Pflaster zu erhalten.

12. Ein Verfahren zur Herstellung eines transdermalen Pflasters mit Rasagilin, wobei das Verfahren die folgenden Schritte umfasst:
a) Hinzufügen einer wirksamen Menge von Rasagilin oder eines pharmazeutisch verträglichen Salzes davon zu einem Lösungsmittel mit einer gewünschten Flüchtigkeit, Zugeben und vollständiges Aufschwellen eines organischen Polymermaterials;
b) Zugeben eines Regulators und gleichzeitig oder danach Zugeben Stoffes zur Erhöhung der transdermalen Absorption, um eine Substratmischung zu erhalten, so dass das Rasagilin oder das pharmazeutisch verträgliche Salz davon in Form einer freien Base vorliegt; und
c) Verschmieren der Substratmischung auf einer inerten Trägerschicht unter Bildung eines gleichmäßigen dünnen Films, und Aufkleben einer Schutzschicht um das Pflaster zu erhalten.

13. Verfahren zur Herstellung eines transdermalen Pflasters mit Rasagilin nach Anspruch 11 oder 12, wobei der Schritt c) ferner das Wiederholen des Verschmierens in irgendeiner Weise umfasst, um ein Substrat mit einem mehrschichtigen dünnen Film zu erhalten.

14. Verfahren zur Herstellung eines transdermalen Pflasters mit Rasagilin nach Anspruch 11 oder 12, wobei der Schritt c) ferner eine Haftschicht frei von Rasagilin als äußerste Schicht umfassen kann.

15. Das transdermale Pflaster mit Rasagilin zur Behandlung oder Prophylaxe von einer Erkrankung des Nervensystems nach Anspruch 1, wobei die Erkrankung des Nervensystems Parkinson-Krankheit, Alzheimer-Krankheit, Depression, kindliche Hyperaktivität, Restless Leg Syndrom, Multiple Sklerose und Entzugserscheinungen umfasst.

## Revendications

1. Timbre transdermique à la rasagiline destiné au traitement ou à la prophylaxie des maladies du système nerveux, dans lequel le timbre comprend une couche de support chimiquement inerte aux ingrédients du substrat, une couche de substrat comprenant de la rasagiline ou un sel pharmaceutiquement acceptable de celle-ci, et une couche protectrice à retirer avant utilisation, dans lequel la couche de substrat est un réservoir porteur de médicament comprenant une matière polymère organique et une matière inorganique ou organique en tant que régulateur, le réservoir comprend de la rasagiline, et la couche de substrat comprend en outre une ou plusieurs substances destinées à améliorer l'absorption transdermique de la rasagiline, dans lequel la rasagiline ou le sel pharmaceutiquement acceptable de celle-ci dans la couche de substrat se présente sous la forme d'une base libre.

2. Timbre transdermique à la rasagiline selon la revendication 1, dans lequel la couche de substrat comprend en outre une couche de substrat à libération contrôlée et/ou une couche adhésive.

3. Timbre transdermique à la rasagiline selon la revendication 1, dans lequel la rasagiline ou le sel pharmaceutiquement acceptable de celle-ci dans la couche de substrat a une quantité efficace de 0,01 mg/cm² à 50 mg/cm².

4. Timbre transdermique à la rasagiline selon l'une quelconque des revendications 1 à 3, dans lequel le sel pharmaceutiquement acceptable de la rasagiline comprend le chlorhydrate, le mésylate, l'ésylate ou le sulfate.

5. Timbre transdermique à la rasagiline selon la revendication 4, dans lequel le sel pharmaceutiquement acceptable de la rasagiline est le mésylate.

6. Timbre transdermique à la rasagiline selon la revendication 1, dans lequel la matière polymère organique comprend au moins un type des matières polymères organiques suivantes : les polyacrylates et les dérivés de ceux-ci, la polyvinylpyrrolidone et les dérivés de celle-ci, les polymères de silicone et les dérivés de ceux-ci, l'alcool polyvinylique et les dérivés de celui-ci, le polyisobutylène et les dérivés de celui-ci, les copolymères d'éthylène-acétate de vinyle et les dérivés de ceux-ci, et le carbopol et les dérivés de celui-ci.

7. Timbre transdermique à la rasagiline selon la revendication 1, dans lequel la matière inorganique ou organique en tant que régulateur comprend une ou plusieurs des substances suivantes : l'eau, l'hydroxyde de sodium, le phosphate de sodium, l'hydrogénophosphate de sodium, le dihydrogénophosphate de sodium, le carbonate de sodium, l'hydrogénocarbonate de sodium, le chlorure de sodium, le sulfate de sodium, l'hydrogénosulfate de sodium, l'hydroxyde de potassium, le phosphate de potassium, l'hydrogénophosphate de potassium, le dihydrogénophosphate de potassium, le carbonate de potassium, l'hydrogénocarbonate de potassium, le chlorure de potassium, le sulfate de potassium, l'hydrogénophosphate de potassium, l'hydroxyde de calcium, le phosphate de calcium, l'hydrogénophosphate de calcium, le carbonate de calcium, le chlorure de calcium, le sulfate de calcium, l'acide phosphorique, l'acide chlorhydrique, l'acide sulfurique, le dioxyde de silicium, l'acide silicique, le métasilicate de sodium, le métasilicate de potassium, le trisilicate de sodium, le trisilicate de potassium, le dioxyde de titane, l'éthanediamine, la triéthylamine, la triéthanolamine, le méthanol, l'éthanol, le propanol, le propylène glycol, le glycérol, le butanol, le chloroforme, le dichlorométhane, le tétrachlorométhane, l'urée, l'éther de pétrole, la polyvidone, le polyéthylène glycol, les alcools gras à chaîne longue, les huiles et les lipides.

8. Timbre transdermique à la rasagiline selon la revendication 1, dans lequel la substance destinée à améliorer l'absorption transdermique de la rasagiline comprend au moins une des substances suivantes : l'éthanol, le propylène glycol, l'acide oléique, l'océnol, l'acide linoléique, le menthol, le laurinol, l'acide laurique, le myristate d'isopropyle, les azacycloheptan-2-ones et les terpènes.

9. Timbre transdermique à la rasagiline selon la revendication 1, dans lequel la matière inorganique ou organique comprend une substance alcaline.

10. Timbre transdermique à la rasagiline selon la revendication 9, dans lequel la substance alcaline est l'hydroxyde de sodium ou la triéthanolamine.

11. Procédé de préparation d'un timbre transdermique à la rasagiline, dans lequel le procédé comprend les étapes suivantes :
a) Mélanger une quantité efficace de rasagiline ou d'un sel pharmaceutiquement acceptable de celle-ci dans une matière polymère organique préchauffée à 50°C à 200°C ;
b) Ajouter un régulateur et une substance destinée à améliorer l'absorption transdermique, mélanger uniformément pour obtenir un substrat de mélange de sorte que la rasagiline ou un sel pharmaceutiquement acceptable de celle-ci dans le substrat se présente sous la forme d'une base libre ; et
c) Enduire le substrat de mélange sur une couche de support inerte pour former une fine pellicule uniforme, et coller dessus une couche protectrice pour obtenir le timbre.

12. Procédé de préparation d'un timbre transdermique à la rasagiline, dans lequel le procédé comprend les étapes suivantes :
a) Ajouter une quantité efficace de rasagiline ou d'un sel pharmaceutiquement acceptable de celle-ci à un solvant ayant une volatilité souhaitée, y ajouter et y faire gonfler entièrement une matière polymère organique ;
b) Y ajouter simultanément ou dans l'ordre un régulateur et une substance destinée à augmenter l'absorption transdermique pour obtenir un substrat de mélange de sorte que la rasagiline ou le sel pharmaceutiquement acceptable de celle-ci se présente sous la forme d'une base libre ; et
c) Enduire le substrat de mélange sur une couche de support inerte pour former une fine pellicule uniforme, et coller dessus une couche protectrice pour obtenir le timbre.

13. Procédé de préparation d'un timbre transdermique à la rasagiline selon la revendication 11 ou 12, dans lequel l'étape c) comprend en outre la répétition de l'application de l'enduit d'une quelconque manière pour obtenir un substrat à pellicule mince multicouche.

14. Procédé de préparation d'un timbre transdermique à la rasagiline selon la revendication 11 ou 12, dans lequel l'étape c) peut comprendre en outre une couche adhésive exempte de rasagiline en tant que couche la plus à l'extérieur.

15. Timbre transdermique à la rasagiline destiné à être utilisé dans le traitement ou la prophylaxie d'une maladie du système nerveux selon la revendication 1, dans lequel la maladie du système nerveux comprend la maladie de Parkinson, la maladie d'Alzheimer, la dépression, le syndrome de l'enfant hyperactif, le syndrome des jambes sans repos, la sclérose en plaques et le syndrome de l'abstinence.
